Europäisches Patentamt

(19) European Patent Office

Office européen des brevets

(11) Publication number: **0 088 495**
**B1**

(12) **EUROPEAN PATENT SPECIFICATION**

(45) Date of publication of patent specification: **11.09.85**

(21) Application number: **83300360.1**

(22) Date of filing: **25.01.83**

(51) Int. Cl.⁴: **C 07 C 1/20, C 07 C 1/24, C 07 C 11/02, B 01 J 29/28**

(54) **Process for converting alcohols into olefins.**

(30) Priority: **05.02.82 US 346430**

(43) Date of publication of application: **14.09.83 Bulletin 83/37**

(45) Publication of the grant of the patent: **11.09.85 Bulletin 85/37**

(84) Designated Contracting States: **BE DE FR GB IT NL**

(56) References cited:
US-A-4 062 905
US-A-4 229 608
US-A-4 251 484
US-A-4 338 475

(73) Proprietor: **MOBIL OIL CORPORATION**
**150 East 42nd Street**
**.New York New York 10017 (US)**

(72) Inventor: **Lee, Wooyoung**
**4 Cohasset Lane**
**Cherry Hill New Jersey 08003 (US)**
Inventor: **Sapre, Ajit Vishwanath**
**3410 Clubhouse Drive**
**West Deptford New Jersey 08066 (US)**
Inventor: **Yurchak, Sergei**
**211 Timber Jump Lane**
**Media Pennsylvania 19063 (US)**

(74) Representative: **West, Alan Harry et al**
**Mobil Court 3 Clements Inn**
**London WC2A 2EB (GB)**

Courier Press, Leamington Spa, England.

# 0 088 495

**Description**

This invention relates to the production of olefins from aliphatic alcohols, and more particularly, to the catalytic conversion of methanol into ethylene.

The ever-increasing demand for olefinic feedstocks has periodically caused a shortage of petrochemical raw materials either because of a limitation in availability of suitable quality petroleum feedstocks from which they have traditionally been derived or a limitation in naphtha cracking capacity. An alternative source of ethylene from non-petroleum sources is one obvious means of keeping pace with the demand for ethylene and other olefins.

It is known that methanol and/or dimethyl ether, which may be obtained from coal, natural gas or biomass, can be converted into more complex hydrocarbons such as olefins and aromatics, by utilizing a group of catalytic zeolites exemplified by ZSM-5 zeolite. Ethylene is one of the olefinic hydrocarbons which may be obtained from such catalytic conversions. The reaction is highly exothermic and the olefins initially formed have a tendency to undergo further reaction to produce aromatic hydrocarbons useful in the production of motor gasoline.

Many patent publications are concerned with various aspects of the conversion of methanol and/or dimethyl ether into light olefins. Thus, the production of olefins from aliphatic ethers by catalytic conversion with a HZSM-5 zeolite is described in U.S. Patent 3,894,106. U.S. Patent 3,979,472 describes the conversion of lower alcohols and their ethers with a composite of antimony oxide and a ZSM-5 zeolite to produce a mixture of ethylene, propylene and mononuclear aromatics. U.S. Patent 4,025,572 discloses that ethylene selectivity can be improved by combining ZSM-5 with an inert diluent while a similar result is achieved, according to U.S. Patent 4,025,575 through use of subatmospheric partial pressure of the feed. Selectivity to ethylene is also improved by employing ZSM-5 zeolite in large crystal form of at least about 1 micron either alone (U.S. Patent 4,025,571) or in combination with added metals (U.S. Patent 4,148,835). Better selectivity is obtained also by interdispersing amorphous silica within the interior of the crystalline structure of the zeolite catalyst (U.S. Patents 4,060,568 and 4,100,219).

Although such processes perform exceptionally well and are unusually effective in converting lower aliphatic alcohols into olefinic hydrocarbons, it has been found that these conversions are exothermic to varying degrees depending on the particular reactant. For example, the amount of heat generated in the conversion of lower alcohols into hydrocarbon products may be estimated to be in the following ranges:

| Alcohol Reactant | Heat produced, kJ per kg of Hydrocarbon Product |
| --- | --- |
| Methanol | 2300-4600 |
| Ethanol | 460-1450 |
| Propanol | 35-840 |

Thus, the conversion of methanol, and to a lesser degree of ethanol, could be considered excessively exothermic in this regard. Furthermore, because of the inherent character and efficiency of the crystalline aluminosilicate zeolite catalysts, the reaction of methanol, and to a lesser degree of ethanol, tends to be self-accelerating, creating excessively hot local regions in the catalyst bed where the reaction tends to go to cmpletion. In an adiabatic catalyst bed reactor, these highly exothermic reactions can result in high catalyst aging rates, and may cause thermal damage to the catalyst. Furthhermore, such high temperatures can cause an undesirable product distribution. Therefore, it is critical in the conversion of methanol into useful products to provide sufficient heat dissipation so that temperatures encountered in any portion of the catalyst bed are restricted within predetermined limits.

Additionally, it is generally good engineering practice to conduct reactant conversions at elevated pressures to utilize more effectively the reactor volume and attendant equipment. With a methanol charge, however, elevated pressures tend to produce increased quantities of 1,2,4,5-tetramethylbenzene (durene), an undesirable by-product, while lower pressures, for example less than 450 kPa favor the production of light olefins.

Various techniques have been employed to control the heat released in such processes: see for example U.S. Patents 3,931,349 (use of light hydrocarbon diluents), 4,052,479 (operating conditions selected to restrict feed conversion to 5—25%) and 4,238,631 (riser reactor and dense fluid catalyst bed). U.S. Patent 4,035,430 describes arranging the catalyst in a series of beds of increasing size with interstage quenching with methanol, dimethyl ether and/or light hydrocarbons for controlling heat. A tubular reactor is disclosed in U.S. Patent 4,058,576 as a means of removing heat during the catalytic conversion of a lower alcohol into olefins. A two-stage conversion is employed with an alcohol dehydration catalyst utilized in the first stage and a ZSM-5 zeolite in a tubular reactor in the second stage; in one embodiment, the ZSM-5 catalyst is located in the tubes of the reactor with a heat-transfer medium passed through the shell-side of the reactor. As the reaction mixture passes through the catalyst, the heat of reaction released within the tubes is transferred to the heat-exchange medium. No details are provided on the configuration of the tubular reactor insofar as it may influence the thermal stability of the reaction.

The present invention is based on the observation that the heat generated in a tubular reactor from the

2

reaction of a lower aliphatic alcohol or a mixture of such an alcohol and its corresponding ether, in the presence of a zeolite catalyst can be effectively removed by providing reactor tubes having a diameter and a length which will transfer the heat from the interior of the tubes at a rate which the shell-side fluid can effectively remove so as to maintain the reaction temperature at a predetermined value.

According to the present invention, there is provided a process for converting an alcohol having from 1 to 3 carbon atoms or a mixture thereof with its corresponding ether into a hydrocarbon product containing an olefin, which comprises passing a feed comprising such an alcohol or alcohol/ether mixture at an elevated temperature over a catalyst comprising a crystalline aluminosilicate zeolite having either (i) a pore size greater than 5 Angstroms, a silica to alumina mole ratio of at least 12 and a constraint index from 1 to 12 or (ii) pores the major dimension of which is less than 6 Angstroms and pore windows of a size provided by 8-membered rings of oxygen atoms, the catalyst being contained within a plurality of cooled tubular reaction zones each having a length and diameter such that the ratio of the rate of change of heat release with respect to temperature in the reaction zones to the rate of change of heat removal with respect to temperature by cooling, is not greater than unity.

Preferably, the feed is the alcohol/ether effluent of a dehydration reaction in which the alcohol, preferably together with water, is passed over a dehyration catalyst at an elevated temperature.

According to the invention, a lower aliphatic alcohol alone or together with its corresponding ether is converted catalytically into olefinic hydrocarbons in a tubular reaction designed to achieve effective control of the heat released during the reaction, particularly during the conversion of the ether and the alcohol into olefinic hydrocarbons. Preferably, the reactants comprise methanol and dimethyl ether and the olefinic product is predomininantly ethylene.

The process of the invention can be considered as an improvement over the process described in U.S. Patent 4,058,576.

The alcohols that may be used in the process of the invention, or more specifically to the first stage of the combination operation, include methanol, ethanol, propanol, and isopropanol. The feed may consist of a relatively pure single alcohol, or a mixture of any of these alcohols. In general, any mixture comprising: methanol; or ethanol; or propanol; or isopropanol is a suitable feed for the first stage of the process. Reactions that produce more than about 230 kJ/kg of total hydrocarbon product, and preferably more than about 460 kJ/kg of hydrocarbon product, at conversion temperature, are considered suitable for the purpose of the invention.

The preferred feed is methanol, including mixtures of methanol and dimethyl ether.

In the first stage of the process the alcohol is contacted with a dehydration catalyst to produce water and a predominantly ether-rich intermediate product. The dehydration catalyst may be any catalyst which results in the intermediate dehydration of the alcohol reactant to form an ether-rich product of higher carbon to oxygen ratio than the feed.

The dehydration reactions that can take place include those that form simple and mixed ethers such as dimethyl ether and diethyl ether. These intermediates may be formed by the intermolecular dehydration of corresponding alcohol reactants, and all of these dehydrations are exothermic. While this dehydration reaction itself is generally known with alumina compositions, such as gamma alumina, other acidic catalysts known in the art are very effective for dehydration.

It will be recognized that with a methanol feed, no intramolecular dehydration is possible, and therefore the dehydration reaction can only proceed exothermically to form, for example, dimethyl ether.

According to a preferred aspect of the invention, the process comprises two sequential stages of catalytic contact in which both stages generate heat. In the first stage, heat generation is limited by restricting the conversion of methanol to approximately an equilibrium mixture comprising dimethyl ether, methanol and water. The conversion product or first stage effluent, because of the generated heat, generally has a temperature of 310 to 400°C and is suitably adjusted to a temperature of 270 to 430°C depending on the nature of the second stage zeolite catalyst, by passing it in indirect heat exchange with a circulating heat exchange fluid. For example, the heat exchange fluid may be water or the methanol reactant passed to the first stage.

The second stage catalytic conversion converts the first stage effluent comprising methanol, dimethyl ether and water into an olefin-rich product. The operation is highly exothermic and occurs rapidly in the presence of certain crystalline zeolites and particularly ZSM-5 type crystalline zeolites and small pore crystalline zeolites.

In general, the ZSM-5 type zeolites used in accordance with the invention are crystalline zeolites having a silica/alumina ratio greater than 12 and a Constraint Index (C.I.) between 1 and 12. These zeolites and their use as conversion catalysts for lower aliphatic alcohols are described in the U.S. patents referred to above, particularly U.S. Patents 3,894,106, 4,025,571, 4,058,576 and 4,148,835.

The preferred zeolites are ZSM-5 type zeolites as exemplified by ZSM-5, ZSM-11, ZSM-12, ZSM-23, ZSM-35 and ZSM-38, ZSM-5 being particularly preferred.

ZSM-5 is described in U.S. Patent 3,702,886; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 is described in U.S. Patent 4,046,859.

Particularly preferred catalysts are those comprising ZSM-5 type zeolite with large crystals, i.e. a crystal size of at least 1 micron, as described in U.S. Patents 4,025,571 and 4,148,835. Another class of particularly preferred catalysts are those comprising ZSM-5 and which contain additional ingredients to improve ethylene selectively, such as amorphous silica interdispersed within the interior of the zeolite crystalline structure. Catalysts of this type are described in U.S. Patents 4,060,568 and 4,100,219.

In addition to the ZSM-5 zeolites, other zeolites known in the art as small pore crystalline aluminosilicate zeolites may be employed in accordance with the invention. These small pore zeolites may be either naturally occurring or synthetic and include, for example, erionite, chabazite, zeolite T, zeolite ZK-5 and ZSM-34. Zeolite T is described in U.S. Patent 2,950,952, zeolite ZK-5 in U.S. Patent 3,427,195, and ZSM-34 in U.S. Patents 4,079,095 and 4,079,096. The crystal structure of this class of zeolites is such as to provide access to and egress from the intracrystalline free space by virtue of the zeolites having pores the major dimension of which is greater than 3 but less than 6 Angstrom units. These zeolites are further characterized by pore windows of about a size such as would be provided by 8-membered rings of oxygen atoms. It will be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. The pores characterizing these zeolites may be substantially circular, such as in zeolite ZK-5 having uniform pores of about 3.9 Angstroms diameter of somewhat elliptical, such as in erionite having pores of approximately 3.6 by 5.2 Angstroms. It will be understood that, in any case, the small pore zeolites have a major pore dimension of less than 6 Angstroms. The pore size dimensions of these zeolites, as well as other feasible zeolites, are those specified in "Zeolite Frameworks" by W. M. Meier, and D. H. Olson appearing in Advances in Chemistry Series, Vol. 101, pages 155—170 (1971).

In accordance with the invention, the conversion into olefinic hydrocarbons is accomplished in catalyst-containing reaction tubes of desired length and diameter to provide a contact time with the catalyst sufficient to accomplish the reaction desired. The reaction zone therefore comprises a plurality of adjacent catalyst-containing tubes of restricted cross-section and limited to provide a desired heat transfer relationship between catalyst particles in the reaction tubes with a temperature controlling liquid medium circulated external to the reaction tube. Thus, the conversion is carried out in a plurality of adjacent, parallel reaction tubes of restricted length and cross-section, each of which is in indirect heat exchange with a heat exchange fluid and sized to provide the required reactant contact time and temperature.

In accordance with the invention, the tubes of the tubular reactor must have a diameter and a length which will permit the heat generated within the tubes to be removed by the heat transfer medium flowing through the shell of the tubular reactor. Where the length and the diameter are not properly specified it is possible that all of the heat generated cannot be expeditiously removed by the heat transfer fluid. This situation can quickly produce an unstable condition where the reaction temperature will escalate in an uncontrolled manner. Stability is achieved when the ratio of the rate of change of heat generation in the tubular reactor with respect to temperature to the rate of change of heat removal by the heat transfer fluid with respect to temperature is no greater than unity, i.e., equal to or less than 1.0. This may be expressed as follows:

$$\phi = \frac{\text{Rate of change of heat generation with temperature}}{\text{Rate of change of heat removal with temperature}}$$

$$\phi = \frac{dQ_g/dT}{dQ_r/dT}$$

where

$Q_g$ = heat generation

$Q_r$ = heat removal

$T$ = temperature

The quantity $\phi$ can be computed at every point along the reactor length for a given sized tube (length and diameter) and specified operating conditions (pressure, inlet temperature, feed composition, etc.). To ensure safe operation it is essential to limit the value of $\phi \leqslant 1$ at any location.

4

The following equation for φ can be derived with algebraic manipulations:

$$\phi = \frac{D_t C_{Ao} (-\Delta H) K_o}{4 T_o U} \cdot \frac{1}{\overline{T}} \left[ \frac{(E/RT_o - \overline{T})}{\overline{T}^2} \cdot c + \frac{d\overline{c}/dz}{d\overline{T}/dz} \right] \exp \frac{-E}{RT_o \overline{T}}$$

$$- \frac{Dt \cdot U_g \cdot \rho_g \cdot c_p}{4 U L} \cdot \frac{d^2\overline{T}/dz^2}{d\overline{T}/dz}$$

for Z from O to dT/dZ=O,

where, $D_t$ = diameter of the tube

$C_{Ao}$ = inlet concentration of the reactant

$\Delta H$ = heat of reaction

$K_o$ = preexponential factor of the rate constant

$T_o$ = inlet temperature

$U$ = overall heat transfer coefficient

$T$ = dimensionless temperature at position Z, $(T/T_o)$

$E$ = activation energy

$R$ = gas constant

$\overline{c}$ = dimensionless concentration at position z, $(c/c_o)$

$Z$ = tube distance variable

$u_g$ = velocity of the gas

$p_g$ = density of the gas

$c_p$ = specific heat of the gas

$L$ = length of the tube

The reactor tube design selected must be satisfactory for accomplishing the heat transfer requirements expected under the reaction conditions desired and occurring at reactant space velocities of 0.5 to 3.0 WHSV. During traverse of the reaction tubes the first stage effluent mixture is converted with a selectivity- and activity-modified crystalline zeolite to produce an olefin-rich product. The olefin-rich product formed is maintained at a temperature of 270 to 370°C during contact with the second stage catalyst. Water or any other suitable heat exchange fluid may be used in indirect heat exchange relationship with the catalyst and reactants in the reaction tubes to remove the generated heat.

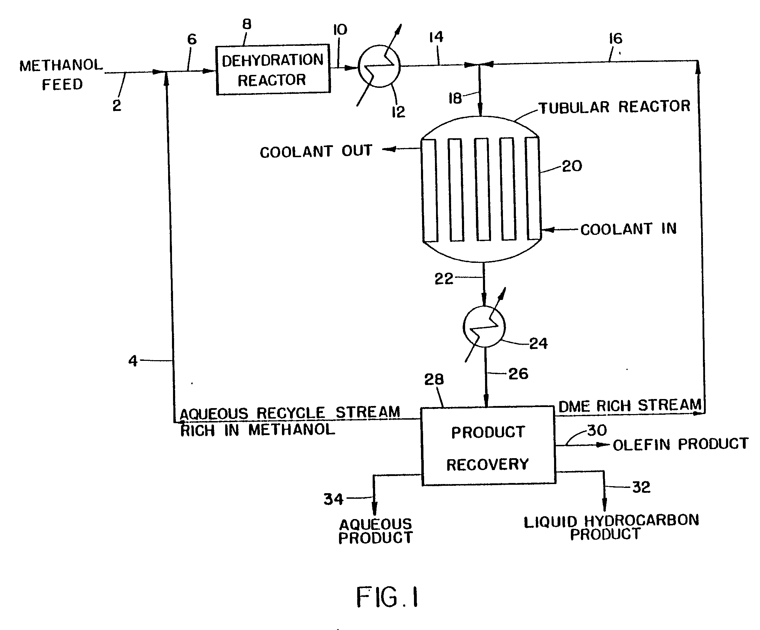

A schematic flow plan of one manner in which the conversion of methanol into a hydrocarbon product rich in ethylene may be achieved in a stable manner in accordance with the present invention is shown by way of example only in Figure 1 of the accompanying drawings. Methanol feed enters through line 2 where it is mixed with an aqueous product recycle stream rich in unreacted methanol from line 4 and passed via line 6 into dehydration reactor 8 where the mixture is converted to an essentially equilibrium mixture of methanol, dimethylether (DME) and water by the catalytic action of a suitable catalyst, such as gamma alumina. This mixture exits the dehydration reactor via line 10 and may undergo either heating or cooling in heat exchanger 12 such that when it flows through line 14 and is combined with unreacted DME from line 16 the resulting mixture passing through line 18 is at the desired temperature for conversion. The combined stream flows through line 18 to tubular reactor 20 which contains a series of 50 mm × 4.5 m tubes packed with ZSM-5 zeolite catalyst. As the mixture passes through the reactor tubes it is converted into an ethylene-rich hydrocarbon stream. The reaction is highly exothermic. The heat released in the tubular reactor is removed by means of a suitable coolant (for example water, molten salt or organic liquid) passing through the shell-side of the reactor. The effluent leaves the reactor through line 22, is cooled in heat exchanger 24, and enters product recovery section 28 via line 26.

The cooled and partially condensed effluent is separated in the product recovery section by known means (not shown) into three phases: (1) a liquid hydrocarbon phase, (2) a liquid aqueous phase containing most of the unreacted methanol and a small amount of DME, and (3) a gas phase rich in light olefins including ethylene and which contains most of the unreacted DME.

The gas phase product may be compressed and sent to an absorber where DME is recovered. The gaseous hydrocarbons can be sent to an olefins recovery facility similar to that employed in conventional olefin plants. Alternatively, the gaseous hydrocarbon product containing DME may be processed by distillation to obtain the desired ethylene product and unreacted DME for recycle to the reactor. The ethylene containing product is recovered through line 30 while the DME is recycled through line 16.

The liquid hydrocarbon phase recovered through line 32 may be stabilized to recover light olefin products. The stabilized liquid hydrocarbon may be blended with gasoline boiling-range components recovered from the olefins recovery facility to make either finished gasoline or a gasoline blending stock.

The aqueous liquid phase may be subject to steam stripping, evaporation, and distillation to recover unreacted methanol and some water for recycle to the dehydration reactor through line 4. Surplus water is removed through line 34. It is not necessary to employ all three separation methods to obtain the desired recycle stream. Any unreacted DME present initially in the aqueous liquid phase will be recovered in the aqueous recycle stream (stream 4). Most of the water product produced by the conversion process in addition to non-chemically bound water present in the methanol feed is removed by stream 34.

The water content of the methanol feed to the process can vary up to 70 weight percent, preferably from 20 to 50 weight percent. Any "equivalent" methanol-water feed may also be employed. A methanol-water feed is said to be "equivalent" to a given methanol-water feed when the methanol-water feed in question plus any appropriate recycle streams recovered from the product of the conversion produces a feed to the zeolite catalyst which has substantially the same composition as the equilibrium mixture obtained when the given methanol-water feed is contacted with a dehydration catalyst.

In Figure 1 the unreacted DME is recycled to the tubular reactor rather than the dehydration reactor. This has the advantage that it allows a higher degree of conversion to be achieved in the dehydration reactor, thus reducing the heat load in the tubular reactor. In addition, since the heat of reaction for converting DME to an olefinic hydrocarbon product is less than that for methanol, feeding unconverted DME directly to the tubular reactor also reduces the heat load on it. Because stable operation of a tubular reactor requires that the rate of heat removal be equal to or somewhat greater than the rate of heat generation, the above scheme allows a tubular reactor to be operated in a stable manner over a wider range of conditions than otherwise possible. Of course, by being more restrictive on the tubular reactor operating conditions, it is possible to operate the dehydration reactor with feed streams 2, 4 and 16.

The following operating conditions may be usefully employed for a tubular reactor which is preceded by a dehydration reactor processing recycle streams in addition to methanol feed. It is well known that high operating pressures result in reduced yields of light olefins. Therefore the inlet pressure to the tubular reactor should be less than 450 kPa, and preferably less than 380 kPa. To provide sufficient heat transfer area with reasonable diameter tubes, the tube length should be 3 to 6 m. Pressure drop and economic considerations indicate that the space velocity (WHSV) to the tubular reactor should be in the range of 0.5—3.0. The operating temperature range for the tubular reactor is 270 to 370°C for a ZSM-5 type zeolite and 315 to 430°C for a small pore zeolite. Compensation for catalyst aging may be made by raising the reactor temperature. It is preferred to use a catalyst made with large crystal (at least 1 micron) ZSM-5 zeolite in the tubular reactor, such as that disclosed in U.S. Patents 4,025,571 and 4,148,835. The ZSM-5 catalyst may be unsteamed or presteamed to reduce its hexane cracking activity (alpha value). Under the operating conditions described here the alpha value should exceed 20. The catalyst may also contain additional ingredients which improve ethylene selectivity, for example intracrystalline silica as described in U.S. Patents 4,060,568 and 4,100,219. Of course, other suitable catalysts may also be employed.

It is preferred to limit the conversion level of methanol and DME in the tubular reactor to between 30 and 90%. At higher conversion levels, significant quantities of the feed stream will be converted into aromatic compounds. In addition to tube diameter, the tube length and the factors listed above, the water content of the feed entering the tubular reactor strongly affects the conversion level which can be achieved while still maintaining stable operation. For example, with a tube size of 38 mm diameter by 4.5 m long, it is possible to operate at 50-80% conversion in a stable manner if the feed to the reactor has a water content equivalent to a 50 wt % methanol/water mixture. If the water content of the feed is decreased to a value equivalent to a 80 wt % methanol/water mixture, the conversion level must be maintained at less than 50% to guarantee stable operation. These restrictions can be relaxed by decreasing the tube diameter.

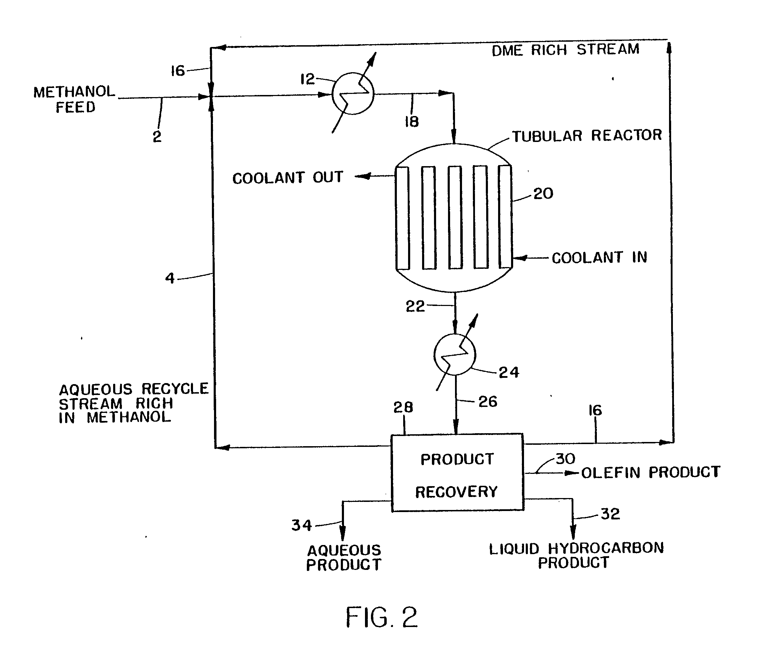

Under certain conditions it is possible to eliminate the dehydration reactor shown in Figure 1. This feature is shown in Figure 2. Since the equipment is similar to that of Figure 1, like numerals are employed in Figure 2 to designate like equipment. Methanol feed in line 2 is mixed with an aqueous recycle stream rich in unreacted methanol from line 4 and a DME-rich stream containing unreacted DME from line 16 to form a combined stream having the water content equivalent to a 50% methanol/water mixture through an 80% methanol/water mixture. The combined streams are heated in heat exchanger 12 and passed into tubular reactor 20 via line 18. The methanol and DME are partially converted in the tubular reactor to a hydrocarbon product rich in ethylene. The reactor effluent leaves the reactor through line 22, is cooled in heat exchanger 24 and sent to product recovery via line 26. The recovery system is the same as that described above with reference to Figure 1.

In the scheme shown in Figure 2, most of the process conditions are the same as for the tubular reactor preceded by a dehydration reactor, except that in the absence of the dehydration reactor, the heat load on the tubular reactor is substantially increased. This can be compensated for by decreased conversion level or by reducing the tube diameter.

The following Examples illustrate the invention.

## Example I

The effect of tube diameter on the stability of a tubular reactor for partial conversion of methanol into an olefinic hydrocarbon product is illustrated in this example.

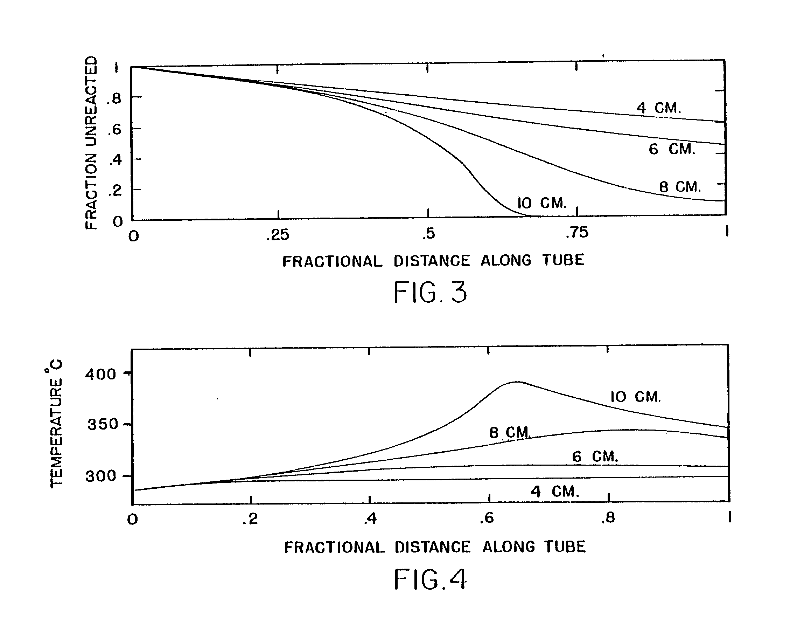

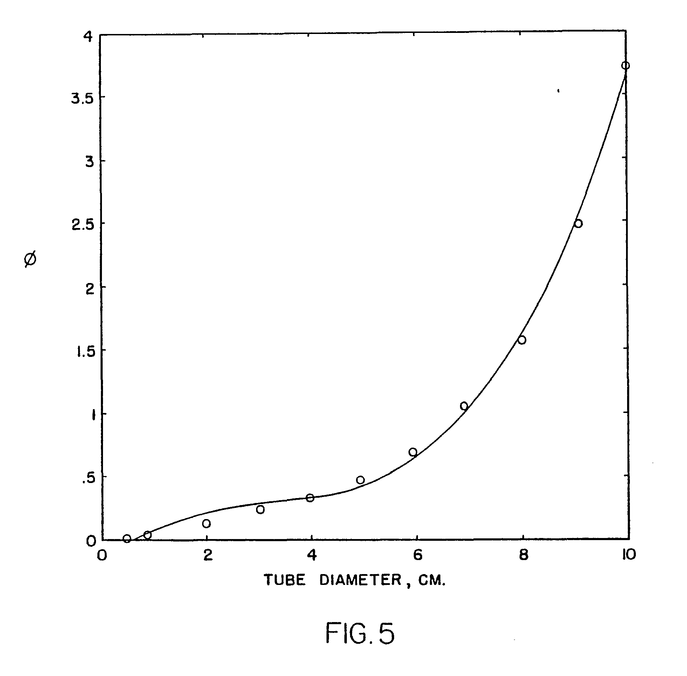

A tubular reactor is used to convert an equilibrium mixture of methanol, dimethylether (DME), and water which is obtained by passing a 50 wt % methanol/water mixture into a dehydration reactor containing a gamma alumina catalyst, for example. The coolant temperature (and reactor feed temperature) is maintained at 293°C. For the particular conditions chosen, the equation φ is employed to evaluate the effect of tube diameter with a constant tube length of 3.66 m. Figure 3 shows the estimated concentration profile of oxygenates (emthanol and DME) in several tubular reactors which differ only in the diameter of the tube. In Figure 3, X is the fractional conversion of the oxygenates. The graph shows that as the tube diameter increases from 4 cm to 10 cm the oxygenate conversion increases and is 100% for the 10 cm tube. Figure 4 shows the estimated temperature profile for the tubular reactors containing different sized tubes. The profile for the 10 cm tube indicates it is impossible to operate this reactor in a stable manner at partial conversion. This information can be expressed in another form as shown in Figure 5. Here the quantity φ is plotted against tube diameter. φ is the ratio of the change of heat generation with respect to temperature by reaction to the rate of change of heat removal by the heat transfer agent with respect to temperature. Values of φ greater than unity are indicative of unstable system. From this plot, it is clear that a tube diameter greater than about 7 cm will result in an unstable reactor. For most purposes, limiting the tube diameter to less than 5 cm for the above feed will provide stable operation over a range of operating conditions.

Example II

Calculating the value of φ for several feed compositions and for varying conversion shows that tube diameter has a significant effect on the thermal stability, as the following table illustrates. Since tube lengths in excess of about 1.8 m have less influence on reactor stability than tube diameter, a tube length of 4.6 m was used in all cases. The reaction can be conducted without a dehydration reactor for the conversion of the methanol-water feed to an equilibrium mixture of methanol-DME and water. In this instance the zeolite catalyst in the tubular reactor is used both for the conversion to the equilibrium mixture and then the reaction to produce the olefinic product. Since both reactions are exothermic, conducting both in the tubular reactor increases the heat load on this reactor. The tubulated vlaues for the tube diameter for stable operation presented below show this difference quite dramatically. The following tabulation provides a comparison of the two cases under otherwise constant conditions.

| Feed to Tubular Reaction (MeOH/Water Equivalent) | Conversion to Hydrocarbons | Tube Diameter of Tubular Reactor if Preceded by | |
| --- | --- | --- | --- |
| | | Dehydration Rx | No Dehydration Rx |
| 50 wt% MeOH/water | 50% | ≤5 cm | ≤3.8 cm |
| 50 wt% MeOH/water | 80% | ≤5 cm | ≤2.5 cm |
| 80 wt% MeOH/water | 50% | ≤3.8 cm | ≤2.5 cm |

These data show that a larger tube diameter (and thus a higher throughput) can be employed to provide stable thermal operation if a separate dehydration reactor is included in the process flow scheme.

**Claims**

1. A process for converting an alcohol having from 1 to 3 carbon atoms or a mixture thereof with its corresponding ether into a hyrocarbon product containing an olefin, which comprises passing a feed comprising such an alcohol or alcohol/ether mixture at an elevated temperature over a catalyst comprising a crystalline aluminosilicate zeolite having either (i) a pore size greater than 5 Angstroms, a silica to alumina mole ratio of at least 12 and a constraint index from 1 to 12 or (ii) pores the major dimension of which is less than 6 Angstroms and pore windows of a size provided by 8-membered rings of oxygen atoms, the catalyst being contained within a plurality of cooled tubular reaction zones each having a length and diameter such that the ratio of the rate of change of heat release with respect to temperature in the reaction zones to the rate of change of heat removal with respect to temperature by cooling, is not greater than unity.

2. A process according to claim 1, wherein the feed also comprises water.

3. A process according to claim 2, wherein the feed is the effluent of a dehydration reaction in which the alcohol is passed over a dehydration catalyst at an elevated temperature.

4. A process according to any one of claims 1 to 3, wherein the alochol is methanol and the ether is dimethyl ether.

5. A process according to any one of claims 1 to 4, wherein the olefin is ethylene.

6. A process according to any one of claims 1 to 5, wherein each tubular reaction zone has a diameter of less than 50 mm.

**Patentansprüche**

1. Verfahren zur Umwandlung eines Alkohols mit von 1 bis 3 Kohlenstoffatomen oder einer Mischung

**0 088 495**

eines solchen Alkohols mit seinem entsprechenden Ether in ein Kohlenwasserstoffprodukt, das ein Olefin enthält, das das Leiten eines Einsatzproduktes, das einen derartigen Alkohol oder eine Alkohol/Ether-Mischung umfaßt, bei einer erhöhten Temperatur über einen Katalysator umfaßt, der einen kristallinen Alumosilikat-Zeolithen, der entweder (i) eine Porengröße von mehr als 5 Angström, ein Siliciumdioxid/Aluminiumoxid-Molverhältnis von wenigstens 12 sowie einen Grenzwertindex (Constraint Index) von 1 bis 12 aufweist oder (ii) Poren aufweist, deren Hauptabmessung weniger als 6 Angström beträgt, und Porenfenster einer Größe aufweist, die von achtgliedrigen Ringen von Sauerstoffatomen erzeugt wird, aufweist, wobei der Katalysator in einer Vielzahl von gekühlten Rohr-Reaktionszonen enthalten ist, die jeweils eine solche Länge und einen solchen Durchmesser aufweisen, daß das Verhältnis der Änderungsgeschwindigkeit der Wärmeabgabe im Hinblick auf die Temperatur in den Reaktionszonen zur Änderungsgeschwindigkeit der Wärmeabführung im Hinblick auf die Temperatur durch Kühlen nicht größer als eine Einheit ist.

2. Verfahren nach Anspruch 1, bei dem das Einsatzprodukt auch Wasser enthält.

3. Verfahren nach Anspruch 2, bei dem das Einsatzprodukt das abströmende Produkt aus einer Wasserabspaltungs-Reaktion ist, bei der der Alkohol bei einer erhöhten Temperatur über einen Wasserabspaltungs-Katalysator geleitet wird.

4. Verfahren nach irgendeinem der Ansprüche 1 bis 3, bei dem der Alkohol Methanol ist und der Ether Dimethyl-ether ist.

5. Verfahren nach irgendeinem der Ansprüche 1 bis 4, bei dem das Olefin Ethylen ist.

6. Verfahren nach irgendeinem der Ansprüche 1 bis 5, bei dem jede Rohr-Reaktionszone einen Durchmesser von weniger als 50 mm aufweist.

**Revendications**

1. Un procédé pour la conversion d'un alcool possédant 1 à 3 atomes de carbone ou d'un mélange de celuici avec son éther correspondant en un produit hydrocarboné contenant une oléfine, ledit procédé consistant à faire passer une charage d'alimentation renfermant un tel alcool ou un mélange alcool/éther, à température élevée, au-dessus d'un catalyseur comprenant une zéolithe à base d'un aluminosilicate cristallin possédant i) soit une dimension de pore supérieure à 5 angströms, un rapport molaire de silice à alumine d'au moins 12 et un indice de contrainte de 1 à 12, ii) soit des pores dont la dimension principale est inférieure à 6 angströms et des ouvertures de pore dont la dimension est fournie par des cycles d'atomes d'oxygène octogonaux, le catalyseur se trouvant dans une pluralité de zones réactionnelles tubulaires refroidies possédant chacune une longueur et un diamètre tels que le rapport du taux de variation de la libération de chaleur en fonction de la température dans les zones réactionnelles au taux de variation de l'extraction de chaleur par refroidissement en fonction de la température ne soit pas supérieur à l'unité.

2. Procédé selon la revendication 1, caractérisé en ce que la charge d'alimentation contient également de l'eau.

3. Procédé selon la revendication 2, caractérisé en ce que la charge d'alimentation est l'effluent d'une réaction de déshydratation au cours de laquelle on fait passer l'alcool sur un catalyseur de déshydratation à température élevée.

4. Procédé selon l'une quelconque des revendications 1 à 3, caractérisé en ce que l'alcool est le méthanol et l'éther est l'éther diméthylique.

5. Procédé selon l'une quelconque des revendications 1 à 4, caractérisé en ce que l'oléfine est l'éthylène.

6. Procédé selon l'une quelconque des revendications 1 à 5, caractérisé en ce que chaque zone réactionnelle tubulaire possède un diamètre de moins de 50 mm.

FIG.I

FIG. 2

FIG. 3

FRACTIONAL DISTANCE ALONG TUBE

FRACTION UNREACTED

4 CM.
6 CM.
8 CM.
IO CM.

FIG. 4

FRACTIONAL DISTANCE ALONG TUBE

TEMPERATURE °C

IO CM.
8 CM.
6 CM.
4 CM.

FIG. 5